# EUROPEAN PATENT SPECIFICATION

(11) **EP 3 331 945 B1**
(45) Date of publication and mention of the grant of the patent: **07.10.2020**
(21) Application number: 16739123.4
(22) Date of filing: 14.07.2016
(51) Int. Cl.: C08K 5/134, C08K 5/00, F16L 9/12, C08K 5/523

(54) **PIPE FOR THE TRANSPORT OF WATER HAVING IMPROVED RESISTANCE TO CHLORINATED DISINFECTANTS**
AUS EINER POLYMERZUSAMMENSETZUNG MIT EINEM POLYOLEFIN HERGESTELLTES ROHR
TUYAU RÉALISÉ AVEC UNE COMPOSITION POLYMÈRE COMPRENANT UNE POLYOLÉFINE

(30) Priority: 04.08.2015 EP 15179628
(43) Date of publication of application: 13.06.2018
(73) Proprietor: SABIC Global Technologies B.V., 4612 PX Bergen op Zoom (NL)
(72) Inventor: BOERAKKER, Mark Johannes, 6160 GA Geleen (NL); VAN MIERLOO, Sarah, 6160 GA Geleen (NL); VOETS, Patrick Elisabeth Luc, 6160 GA Geleen (NL)
(74) Representative: Sabic Intellectual Property Group
(86) International application number: PCT/EP2016/066795
(87) International publication number: WO 2017/021124

(56) References cited:
- DE-A1- 19 629 429
- US-A1- 2011 144 246
- US-A1- 2013 035 426
- US-A1- 2013 122 228
- US-A1- 2014 163 145
- US-A1- 2015 090 671

## Description

The present invention relates to a pipe for the transport of water produced with a polymer composition comprising ; multimodal polyethylene. The pipe has improved resistance to chlorinated disinfectants.

Pipes for the transport of gas, for sanitation and for water supply may be produced with for example bimodal polyethylene compositions. Pipes have a very good resistance to water however their lifetime is shortened when the pipes come into contact with disinfectants which are often added to water for hygienic reasons. The chlorine dioxide used as disinfectant in water degrades most materials including polyethylene (Colin, Aging of polyethylene pipes transporting drinking water disinfected by chlorine dioxide, part I, Chemical aspects; Polymer engineering and Science 49(7); 1429-1437; July 2009). Other chlorinated solvents are for example chloramine and chlorine. It is known in the art to apply additives for example antioxidants and stabilizers to prevent said degradation. Several types of additives are proposed to protect polymers during processing and to achieve the desired end-use properties. However, appropriate combinations of stabilizers have to be carefully selected, depending on the desired final properties the polymeric article should have.

It is the object of the present invention to provide a pipe with improved service lifetime for the transportation of water containing chlorinated disinfectants, for example chlorine dioxide, chloramine and chlorine.

The pipe for the transport of water with resistance to chlorinated disinfectants according to the invention is produced with a polymer composition comprising multimodal polyethylene and a bisphenol monoester represented by Formula 1: 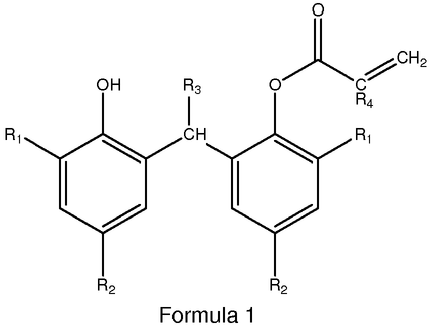 wherein each R₁ and R₂ independently represents an alkyl group having 1 to 5 carbon atoms, R₃ represents a hydrogen atom or an alkyl group having 1 to 3 carbon atoms, and R₄ represents a hydrogen atom or a methyl group or by Formula 2: 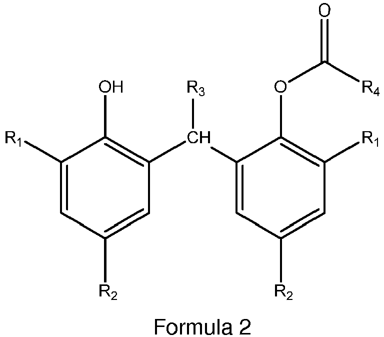 wherein each R₁ and R₂ independently represents an alkyl group having 1 to 5 carbon atoms, R₃ represents a hydrogen atom or an alkyl group having 1 to 3 carbon atoms, and R₄ group represents vinyl, vinyl ether or vinyl amine, 1-alkadiene, 1-alkene having 1 to 15 carbon atoms.

The drinking water pipe, preferably a pressure pipe, based on the polyethylene composition according to the invention has an improved protection against for example chlorine dioxide containing cold or hot water and consequently a longer life time. It is also possible to transport waste water or cooling water.

Preferably the amount of polyethylene in the composition is higher than 95.0 wt%.

Preferably the amount of bisphenol monoester in the composition is lower than 1.0 wt% and higher than 0.05 wt%.

More preferably the amount of bisphenol monoester in the composition is lower than of lower than 0.6 wt%.

Most preferably the amount of bisphenol monoester in the composition ranges between 0.05 and 0.4 wt%. This amount protects the pipe against chlorine dioxide during a long period.

The multimodal polyethylene may be for example a bimodal or trimodal polyethylene or polypropylene.

Preferably, the multimodal polyethylene is bimodal polyethylene.

According to a preferred embodiment of the invention the pipe according to the invention produced with a polymer composition comprising multimodal polyethylene and a bisphenol monoester represented by Formula 1: 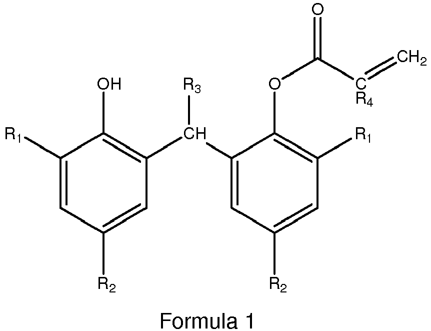 wherein each R₁ and R₂ independently represents an alkyl group having 1 to 5 carbon atoms, R₃ represents a hydrogen atom or an alkyl group having 1 to 3 carbon atoms, and R₄ represents a hydrogen atom or a methyl group.

In Formula 1 R₁ and R₂ independently represents an alkyl group having 1 to 5 carbon atoms. Suitable examples of the alkyl group include a methyl group, an ethyl group, an n-propyl group, an isopropyl group, an n-butyl group, an isobutyl group, a sec-butyl group, a tert-butyl group, an n-pentyl group, a tert-pentyl group and the like. In particular, R₁ is preferably an alkyl group having a tertiary carbon, that is, a tert-butyl group or a tert-pentyl group.

In Formula 1 R₃ represents a hydrogen atom or an alkyl group having 1 to 3 carbon atoms. Examples of the alkyl group include a methyl group, an ethyl group, an n-propyl group, and an isopropyl group, and among these, a methyl group is particularly preferable.

Suitable examples of the bisphenol monoester to be used in the present invention include 2-[1-(2-hydroxy-3,5-di-tert-pentylphenyl)ethyl]-4,6-di-tert-pentylphenylacrylate , 2,4-di-tert-pentyl-6-[1-(3,5-di-tert-pentyl-2-hydroxyphenyl) ethyl]phenylacrylate, 2-tert-butyl-6-[1-(3-tert-butyl-2-hydroxy-5-methylphenyl)methyl]-4-methylphenylacrylate, 2-[1-(2-hydroxy-3,5-di-tert-pentylphenyl)ethyl]-4,6-di-tert-pentylphenylmethacrylate, 2,4-di-tert-butyl-6-[1-(3,5-di-tert-butyl-2-hydroxyphenyl)ethyl]phenylacrylate, 2,4-di-tert-butyl-6-[1-(3,5-di-tert-butyl-2-hydroxyphenyl)ethyl]phenylmethacrylate, 2-tert-butyl-6-[1-(3-tert-butyl-2-hydroxy-5-methylphenyl)ethyl]-4-methylphenylacrylate, 2-tert-butyl-6-[1-(3-tert-butyl-2-hydroxy-5-methylphenyl)propyl]-4-methylphenylacrylate, 2-tert-butyl-6-[1-(3-tert-butyl-2-hydroxy-5-propylphenyl)ethyl]-4-propylphenylacrylate and 2-tert-butyl-6-[1-(3-tert-butyl-2-hydroxy-5-isopropylphenyl)ethyl]-4-isopropylphenylacrylate.

According to a preferred embodiment of the invention the bisphenol monoester is selected from 2,4-di-tert-pentyl-6-[1-(3,5-di-tert-pentyl-2-hydroxyphenyl)ethyl]phenylacrylate or 2-[1-(2-hydroxy-3,5-di-tert-pentylphenyl)ethyl]-4,6-di-tert-pentylphenylacrylate and 2-tert-butyl-6-[1-(3-tert-butyl-2-hydroxy-5-methylphenyl)methyl]-4-methylphenylacrylate.

According to a further preferred embodiment of the invention the bisphenol monoester is 2-[1-(2-hydroxy-3,5-di-tert-pentylphenyl)ethyl]-4,6-di-tert-pentylphenylacrylate.

It is possible to apply compounds comprising more than one group according to Formula (1) and/or Formula (2).

According to a further preferred embodiment of the invention the pipe is produced with a composition comprising
(a) bimodal polyethylene
(b) bisphenol monoester
(c) polyphenolic compound and/or
(d) organic phosphite and/or phosphonate
wherein the weight ratio (b): (c+d) ranges between 7:1 and 1:7.

Suitable polyphenolic compounds include for example tetrakis[methylene-3-(3',5'-di-t-butyl-4-hydroxyphenyl)propionate] methane;1,1,3-tris(2-methyl-4-hydroxy-5-t-butylphenyl)butane; 1,3,5-trimethyl-2,4,6-tris(3,5-di-t-butyl-4-hydroxybenzyl)benzene, bis(3,3-bis(4'-hydroxy-3'-t-butylphenyl)butanoic acid]-glycol ester; tris(3,5-di-t-butyl-4-hydroxy benzyl)isocyanurate; 1,3,5-tris(4-t-butyl-2,6-dimethyl-3-hydroxybenzyl)isocyanurate; 5-di-t-butyl-4-hydroxy-hydrocinnamic acid triester with 1,3,5-tris(2-hydroxyethyl)-s-triazine-2,4,6(1H, 3H, 5H)-trione; p-cresol/ dicyclopentadiene butylated reaction product; 2,6-bis(2'-bis-hydroxy-3'-t-butyl-5'-methyl-phenyl-4-methyl-phenol).

A preferred polyphenolic compound is 1,3,5-trimethyl-2,4,6-tris(3,5-di-t-butyl-4-hydroxybenzyl)benzene (Irganox 1330 supplied by BASF).

Suitable organic phosphites and phosphonites include for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphate, bis(2,6-di-tert-butyl-4-methylphenyl) pentaerythritol diphosphate, bisisodecyloxy-pentaerythritol diphosphite, bis(2,4-di-tert-butyl- 6-methylphenyl) pentaerythritol diphosphite, bis(2,4,6-tri-tert-butylphenyl) pentaerythritol diphosphite, tristearyl sorbitol triphosphite, tetrakis(2,4-di-tert-butylphenyl) 4,4'- biphenylenediphosphonite, 6-isooctyloxy-2,4,8,10-tetra-tert-butyl-12H-dibenzo[d,g]-1,3,2-dioxaphosphocin, 6-fluoro-2,4,8,10-tetra-tert-butyl-12-methyldibenzo[d,g]-1,3,2-dioxaphosphocin, bis(2,4-di-tert-butyl-6-methylphenyl) methyl phosphite, bis(2,4-di-tert-butyl-6-methylphenyl) ethyl phosphite.

A preferred phosphite is tris(2,4-di-tert-butylphenyl) phosphite (Irgafos 168 supplied by BASF).

According to another preferred embodiment of the invention the pipe is produced with a composition comprising
(a) multimodal polyethylene
(b) bisphenol monoester
(c) polyphenolic compound and/or
(d) organic phosphite and/or phosphonate
wherein the weight ratio (b): (c+d) ranges between 7:1 and 1:7.

Preferably (b, (c) and (d) are added during the granulation step of the multimodal, for example bimodal, high density polyethylene powder.

According to a preferred embodiment of the invention the components are added to the polyethylene resin while the polyethylene is in a molten state during extrusion.

The components may be added together and may be added separately.

Preferably the components are added in one step.

The multimodal ethylene polymer may be an ethylene homo- or copolymer.

The multimodal ethylene grades to be applied in pipe applications may comprise additives such as for example carbon black, pigments, stearates, a UV stabilizer for example a sterically hindered amine, fillers, minerals ,lubricants and/or other stabilisers.

The production processes for bimodal high density polyethylene (HDPE) are summarised at pages 16-20 of "PE 100 Pipe systems" (edited by Bromstrup; second edition, ISBN 3-8027-2728-2).

The production of bimodal high density polyethylene (HDPE) via a low pressure slurry process is described by Alt et al. in "Bimodal polyethylene-Interplay of catalyst and process" (Macromol.Symp. 2001, 163, 135-143). Bimodal high density polyethylene may be produced via a low pressure slurry process for the production of comprising a polymerisation stage, a powder drying stage, an extrusion and pellet handling stage, a recycling stage and a wax removal unit. In a two stage cascade process the reactors may be fed continuously with a mixture of monomers, hydrogen, catalyst/co-catalyst and diluent recycled from the process. In the reactors, polymerisation of ethylene occurs as an exothermic reaction at pressures in the range between for example 0.5 MPa (5 bar) and 1MPa (10 bar) and at temperatures in the range between for example 75 °C and 88 °C. The heat from the polymerisation reaction is removed by means of cooling water. The characteristics of the polyethylene are determined amongst others by the catalyst system and by the concentrations of catalyst, co monomer and hydrogen. The production of bimodal high density polyethylene (HDPE) via a low pressure slurry process may also be performed via a three stage process.

The concept of the two stage cascade process is elucidated at pages 137-138 by Alt et al. "Bimodal polyethylene-Interplay of catalyst and process" (Macromol. Symp. 2001, 163). The reactors are set up in cascade with different conditions in each reactor including low hydrogen content in the second reactor. This allows for the production of HDPE with a bimodal molecular mass distribution and defined co monomer content in the polyethylene chains.

Suitable catalysts for the production of multimodal polyethylene include Ziegler Natta catalysts, chromium based catalysts and single site metallocene catalysts. In all potential possible technologies the process and the catalyst have to form a well-balanced system. The catalyst is crucial for the polymerisation reaction of multimodal polyethylene. By cooperation of process and catalyst a definite polymer structure is produced.

US20130035426 discloses a composition comprising a specific compound represented by a very broad formula, trehalose and thermoplastic polymer selected from 87 polymers to improve process stability. US20130035426 is not related to a pipe with improved service lifetime for the transportation of water containing chlorinated disinfectants.

DE19629429 is directed to a process to produce grafted polyolefins in the presence of silane compounds which may comprise bisphenol monoesters. DE19629429 provides a solution for problems with respect to a graft process in the presence of siloxane bridges. DE19629429 is not related to a pipe with improved service lifetime for the transportation of water containing chlorinated disinfectants.

The invention will be elucidated by means of the following examples.

### Examples

SABIC Vestolen A5924 (Resin A) used as base polymer in all examples was a bimodal high density polyethylene with MFR₅ of 0.24 g/10min and density 958 kg/m3.

### Examples I-II and Comparative Examples A-C

The Examples I-II and Comparative Examples A-C use different additive packages in combination with Resin A to protect the polyethylene from attack by chlorine dioxide (see Table 1). The components as indicated in Table 1 were mixed at 245 degrees Celcius using a twin screw extruder.

**Table 1**

| Composition | Resin A wt% | Calcium stearate ppm | Carbon black wt% | Irganox 1010 ppm | Irgafos 168 ppm | Bisphenol monoester ppm | Irganox 1330 ppm | DHT4A ppm |
|---|---|---|---|---|---|---|---|---|
| A | 97 | 2000 | 2.5 | 2000 | 1000 | 0 | 0 | 0 |
| B | 96.15 | 2000 | 2.5 | 2000 | 2500 | 0 | 5000 | 2000 |
| C | 96.44 | 2000 | 2.5 | 2000 | 2000 | 0 | 3200 | 1400 |
| I | 96 | 2000 | 2.5 | 2000 | 2500 | 1500 | 5000 | 2000 |
| II | 96.34 | 2000 | 2.5 | 2000 | 2000 | 1000 | 3200 | 1400 |

wherein:
- Irganox 1010 :Tetrakis [methylen-3-(3',5')-di-t-butyl-4'-hydroxyphenyl) propionate] methane commercially available from Ciba Speciality Chemicals,
- Bisphenol monoester: 2-[1-(2-Hydroxy-3,5-di-tert-pentylphenyl)ethyl]-4,6-di-tert-pentylphenyl acrylate;
- Irganox 1330 : 1,3,5-Trimethyl-2,4,6-tris(3,5-di-tert-butyl-4-hydroxybenzyl)benzene;
- Irgafos 168: Tris(2,4-di-tert-butylphenyl) phosphite ;
- DHT-4A®, commercially available hydrotalcite from Kisuma Chemicals.
- Resin A: SABIC Vestolen A5924; bimodal high density polyethylene with MFR₅ of 0.24 g/10min and density 958 kg/m3.

Compounds were compression molded using ISO1872-2 resulting in plaques, which were cut to ISO527-1A tensile bars (4 mm thick).

### Ageing test

The tensile bars were aged in a continuous water flow at a temperature of 40 ºC with a chlorine dioxide concentration maintained at 1 mg/L and a pH maintained at 7.2. Flow rate was regulated at 200 L/h. Water hardness was regulated to 20 ºF. A constant fresh water flow was added during testing allowing full renewal of the testing water each 4 hrs. The compression molded samples were aged for 1000 hrs.

Tensile tests according to Plastics- Determination of tensile properties ISO527-1 at room temperature at a strain rate of 50 mm/min on aged and non-aged tensile bars were performed to determine the residual elongation at break for the aged samples and reported in Table 2.

**Table 2**

| Composition | Elongation @ break before ageing in % | Elongation @ break after ageing in % |
|---|---|---|
| A | 466 | 33 |
| B | 301 | 259 |
| C | 463 | 32 |
| I | 463 | 462 |
| II | 346 | 136 |

From Table 2 it can be concluded that Examples I and II demonstrate significantly higher elongation at break after being exposed to water containing chlorine dioxide than Comparative Example A.

### Comparing

- Comparative Example B to Example I and
- Comparative Example C to Example II
shows that the effect of adding bisphenol monoester had an additional profound effect on the elongation at break as obtained after exposure to water containing chlorine dioxide.

## Claims

1. Pipe for the transport of water with resistance to chlorinated disinfectants produced with a polymer composition comprising polyethylene and a bisphenol monoester represented by Formula 1: 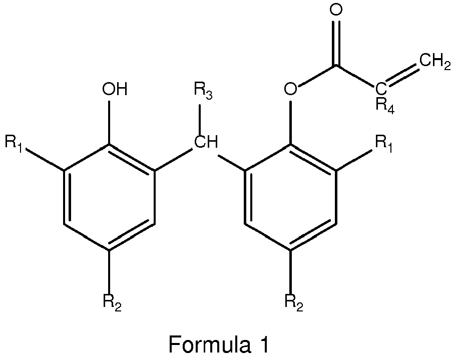 wherein each R₁ and R₂ independently represents an alkyl group having 1 to 5 carbon atoms, R₃ represents a hydrogen atom or an alkyl group having 1 to 3 carbon atoms, and R₄ represents a hydrogen atom or a methyl group or by Formula 2: 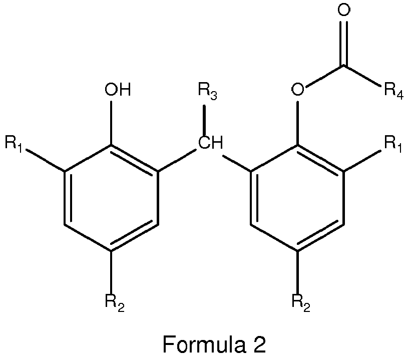
wherein each R₁ and R₂ independently represents an alkyl group having 1 to 5 carbon atoms, R₃ represents a hydrogen atom or an alkyl group having 1 to 3 carbon atoms, and R₄ group represents vinyl, vinyl ether or vinyl amine, 1-alkadiene, 1-alkene having 1 to 15 carbon atoms,
wherein the polyethylene is multimodal polyethylene.

2. Pipe according to claim 1 **characterised in that** the bisphenol monoester is a bisphenol monoester according to Formula 1.

3. Pipe according to any one of Claims 1-2 **characterised in that** the composition comprises a polyphenolic compound and/or an organic phosphite and/or phosphonite.

4. Pipe according to any one of Claims 1-3 **characterised in that** the amount of bisphenol monoester in the composition is lower than 1.0 wt%.

5. Pipe according to Claim 4 **characterised in that** the amount of bisphenol monoester in the composition is lower than 0.6 wt%.

6. Pipe according to Claim 5 **characterised in that** the amount of bisphenol monoester in the composition ranges between 0.05 and 0.4 wt%.

7. Pipe according to Claim 1 **characterised in that** multimodal polyethylene is bimodal polyethylene.

8. Pipe according to any one of Claims 1-7 **characterised in that** the bisphenol ester is selected from the bisphenol monoester is selected from 2,4-di-tert-pentyl-6-[1-(3,5-di-tert-pentyl-2-hydroxyphenyl)ethyl]phenylacrylate or 2-[1-(2-hydroxy-3,5-di-tert-pentylphenyl)ethyl]-4,6-di-tert-pentylphenylacrylate, and 2-tert-butyl-6-[1-(3-tert-butyl-2-hydroxy-5-methylphenyl)methyl]-4-methylphenylacrylate.

9. Pipe according to Claim 8 **characterised in that** the bisphenol ester is 2-[1-(2-hydroxy-3,5-di-tert-pentylphenyl)ethyl]-4,6-di-tert-pentylphenylacrylate.

10. Pipe according to any one of Claims 3-9 **characterised in that** the polyphenolic compound is 1,3,5-trimethyl-2,4,6-tris(3,5-di-t-butyl-4-hydroxybenzyl)benzene

11. Pipe according to any one of Claims 3-10 **characterised in that** the phosphite is tris(2,4-di-tert-butylphenyl) phosphite.

12. Pipe according to any one of Claims 1-11 produced with a composition comprising
(a) polyethylene, wherein the polyethylene is multimodal polyethylene
(b) bisphenol monoester
(c) polyphenolic compound and/or
(d) organic phosphite and/or phosphonate
wherein the weight ratio (b): (c+d) ranges between 7:1 and 1:7.

13. Pipe according to Claim 12 produced with a composition comprising
(a) bimodal polyethylene
(b) bisphenol monoester
(c) polyphenolic compound and/or
(d) organic phosphite and/or phosphonate
wherein the weight ratio (b): (c+d) ranges between 7:1 and 1:7

## Patentansprüche

1. Rohrleitung für den Transport von Wasser mit einer Widerstandsfähigkeit gegen chlorierte Desinfektionsmittel, die mit einer Polymerzusammensetzung hergestellt werden, die Polyethylen und einen Bisphenolmonoester umfasst, dargestellt durch Formel 1: 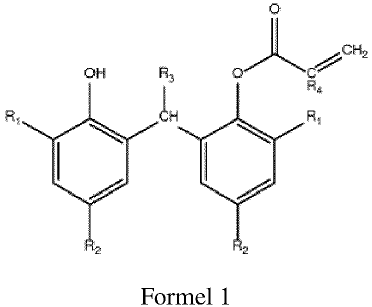 wobei jeweils R₁ und R₂ unabhängig eine Alkylgruppe mit 1 bis 5 Kohlenstoffatomen darstellen, R₃ ein Wasserstoffatom oder eine Alkylgruppe mit 1 bis 3 Kohlenstoffatomen darstellt und R₄ ein Wasserstoffatom oder eine Methylgruppe darstellt, oder durch Formel 2: 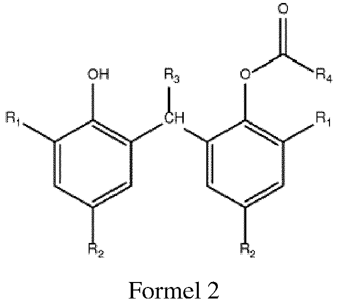
wobei jeweils R₁ und R₂ unabhängig eine Alkylgruppe mit 1 bis 5 Kohlenstoffatomen darstellen, R₃ ein Wasserstoffatom oder eine Alkylgruppe mit 1 bis 3 Kohlenstoffatomen darstellt und die R4-Gruppe Vinyl, Vinylether oder Vinylamin, 1-Alkadien, 1-Alken mit 1 bis 15 Kohlenstoffatomen darstellt,
wobei das Polyethylen ein multimodales Polyethylen ist.

2. Rohrleitung nach Anspruch 1, **dadurch gekennzeichnet, dass** der Bisphenolmonoester ein Bisphenolmonoester gemäß Formel 1 ist.

3. Rohrleitung nach einem der Ansprüche 1 bis 2, **dadurch gekennzeichnet, dass** die Zusammensetzung eine polyphenolische Verbindung und/oder ein organisches Phosphit und/oder Phosphonit umfasst.

4. Rohrleitung nach einem der Ansprüche 1 bis 3, **dadurch gekennzeichnet, dass** die Menge des Bisphenolmonoesters in der Zusammensetzung weniger als 1,0 Gew.-% beträgt.

5. Rohrleitung nach Anspruch 4, **dadurch gekennzeichnet, dass** die Menge des Bisphenolmonoesters in der Zusammensetzung weniger als 0,6 Gew.-% beträgt.

6. Rohrleitung nach Anspruch 5, **dadurch gekennzeichnet, dass** die Menge des Bisphenolmonoesters in der Zusammensetzung im Bereich zwischen 0,05 und 0,4 Gew.-% liegt.

7. Rohrleitung nach Anspruch 1, **dadurch gekennzeichnet, dass** das multimodale Polyethylen bimodales Polyethylen ist.

8. Rohrleitung nach einem der Ansprüche 1 bis 7, **dadurch gekennzeichnet, dass** der Bisphenolester ausgewählt ist aus dem Bisphenolester, das ausgewählt ist von 2,4-Di-tert-pentyl-6-[1-(3,5-di-tert-pentyl-2-hydroxyphenyl)ethyl]phenylacrylat oder 2-[1-(2-Hydroxy-3,5-di-tert-pentylphenyl)ethyl]-4,6-di-tert-pentylphenylacrylat und 2-Tert-butyl-6-[1-(3-tert-butyl-2-hydroxy-5-methylphenyl)methyl]-4-methylphenylacrylat.

9. Rohrleitung nach Anspruch 8, **dadurch gekennzeichnet, dass** der Bisphenolester 2-[1-(2-Hydroxy-3,5-di-tert-pentylphenyl)ethyl]-4,6-di-tert-pentylphenylacrylat ist.

10. Rohrleitung nach einem der Ansprüche 3 bis 9, **dadurch gekennzeichnet, dass** die polyphenolische Verbindung 1,3,5-Trimethyl-2,4,6-tris(3,5-di-t-butyl-4-hydroxybenzyl)benzen ist.

11. Rohrleitung nach einem der Ansprüche 3 bis 10, **dadurch gekennzeichnet, dass** das Phosphit Tris(2,4-di-tert-butylphenyl)phosphit ist.

12. Rohrleitung nach einem der Ansprüche 1 bis 11, hergestellt mit einer Zusammensetzung, die umfasst:
(a) Polyethylen, wobei das Polyethylen multimodales Polyethylen ist
(b) Bisphenolmonoester
(c) polyphenolische Verbindung und/oder
(d) organisches Phosphit und/oder Phosphat
wobei das Gewichtsverhältnis (b):(c+d) im Bereich zwischen 7:1 und 1:7 liegt.

13. Rohrleitung nach Anspruch 12, hergestellt mit einer Zusammensetzung, die umfasst:
(a) Bimodales Polyethylen
(b) Bisphenolmonoester
(c) polyphenolische Verbindung und/oder
(d) organisches Phosphit und/oder Phosphat
wobei das Gewichtsverhältnis (b):(c+d) im Bereich zwischen 7:1 und 1:7 liegt.

## Revendications

1. Tuyau pour le transport d'eau avec résistance à des désinfectants chlorés produit avec une composition de polymère comprenant du polyéthylène et un monoester de bisphénol représenté par la formule 1 : 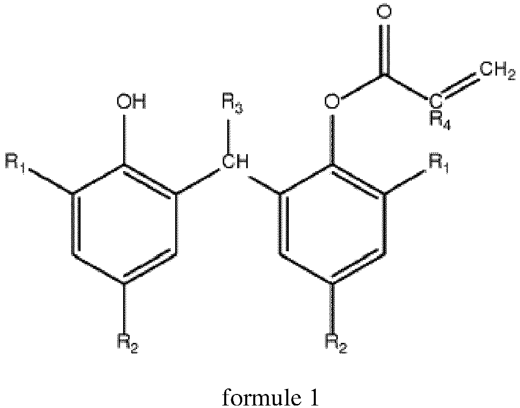 dans lequel chaque R₁ et R₂ représente indépendamment un groupe alkyle ayant d'1 à 5 atomes de carbone, R₃ représente un atome d'hydrogène ou un groupe alkyle ayant d'1 à 3 atomes de carbone, et R₄ représente un atome d'hydrogène ou un groupe méthyle, ou par la formule 2 : 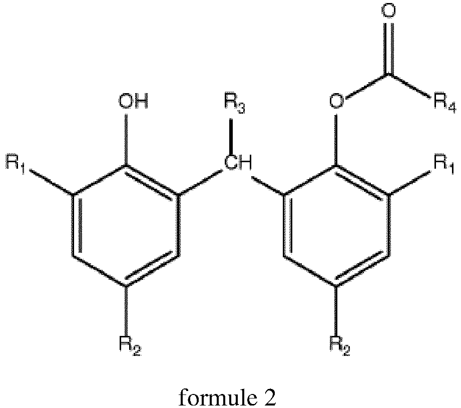
dans lequel chaque R₁ et R₂ représente indépendamment un groupe alkyle ayant d'1 à 5 atomes de carbone, R₃ représente un atome d'hydrogène ou un groupe alkyle ayant d'1 à 3 atomes de carbone, et le groupe R₄ représente : vinyle, éther de vinyle ou amine de vinyle, 1-alkadiène, 1-alcène ayant d'1 à 15 atomes de carbone,
dans lequel le polyéthylène est du polyéthylène multimodal.

2. Tuyau selon la revendication 1, **caractérisé en ce que** le monoester de bisphénol est un monoester de bisphénol selon la formule 1.

3. Tuyau selon l'une quelconque des revendications 1 et 2, **caractérisé en ce que** la composition comprend un composé polyphénolique et/ou un phosphite et/ou phosphonite organique.

4. Tuyau selon l'une quelconque des revendications 1 à 3, **caractérisé en ce que** la quantité de monoester de bisphénol dans la composition est inférieure à 1,0 % en poids.

5. Tuyau selon la revendication 4, **caractérisé en ce que** la quantité de monoester de bisphénol dans la composition est inférieure à 0,6 % en poids.

6. Tuyau selon la revendication 5, **caractérisé en ce que** la quantité de monoester de bisphénol dans la composition varie entre 0,05 et 0,4 % en poids.

7. Tuyau selon la revendication 1, **caractérisé en ce que** le polyéthylène multimodal est du polyéthylène bimodal.

8. Tuyau selon l'une quelconque des revendications 1 à 7 **caractérisé en ce que** l'ester de bisphénol est sélectionné parmi le monoester de bisphénol est sélectionné parmi : 2,4-di-tert-pentyl-6-[1-(3,5-di-tert-pentyl-2-hydroxyphényl)éthyl]phénylacrylate ou 2-{1-(2-hydroxy-3,5-di-tert-pentylphényl)éthyl]-4,6-di-tert-pentylphénylacrylate, et 2-tert-butyl-6-[1-(3-tert-butyl-2-hydroxy-5-méthylphényl)méthyl]-4-méthylphénylacrylate.

9. Tuyau selon la revendication 8, **caractérisé en ce que** l'ester de bisphénol est 2-[1-(2-hydroxy-3,5-di-tert-pentylphényl)éthyl]-4,6-di-tert-pentylphénylacrylate.

10. Tuyau selon l'une quelconque des revendications 3 à 9, **caractérisé en ce que** le composé polyphénolique est du 1,3,5-triméthyl-2,4,6-tris(3,5-di-t-butyl-4-hydroxybenzyl)benzène.

11. Tuyau selon l'une quelconque des revendications 3 à 10, **caractérisé en ce que** le phosphite est du tris(2,4-di-tert-butylphényl)phosphite.

12. Tuyau selon l'une quelconque des revendications 1 à 11, produit avec une composition comprenant
(a) du polyéthylène, dans lequel le polyéthylène est du polyéthylène multimodal
(b) monoester de bisphénol
(c) composé polyphénolique et/ou
(d) phosphite et/ou phosphonate organique
dans lequel le rapport pondéral (b) : (c+d) varie entre 7 : 1 et 1 : 7.

13. Tuyau selon la revendication 12, produit avec une composition comprenant
(a) polyéthylène bimodal
(b) monoester de bisphénol
(c) composé polyphénolique et/ou
(d) phosphite et/ou phosphonate organique
dans lequel le rapport pondéral (b) : (c+d) varie entre 7 : 1 et 1 : 7.
